# EUROPEAN PATENT APPLICATION

(11) **EP 4 678 217 A1**
(43) Date of publication of application: **14.01.2026**
(21) Application number: 23928587.7
(22) Date of filing: 20.03.2023
(51) Int. Cl.: A61M 25/10

(54) **DUAL FILM BALLOON CATHETER**

(71) Applicant: National University Corporation Kobe University, Kobe-shi, Hyogo 657-8501 (JP)
(72) Inventor: KONISHI, Akihide, Kobe-shi, Hyogo 657-8501 (JP); YAGI, Takahiro, Kusatsu-shi, Shiga 525-8577 (JP)
(74) Representative: Fleck, Hermann-Josef
(86) International application number: PCT/JP2023/010980
(87) International publication number: WO 2024/195004

(57) **Abstract**

Provided is a balloon catheter including a single inflation lumen, capable of preventing balloon rupture, and having an increased deflation response. In a bonded part 12a on a front end side, an inner balloon 3 is bonded to an inner tube 5 and an outer balloon 2 is bonded to the inner balloon 3. In a bonded part 12b on a base end side, the inner balloon 3 is bonded to an outer tube 4 and the outer balloon 2 is bonded to the inner balloon 3, thereby forming a balloon catheter having a single inflation lumen 8. The outer balloon 2 and the inner balloon 3 are not bonded in an inflation part 11. The maximum diameter φ₂ of the inner balloon 3 is set such as to be smaller than the maximum diameter φ₁ of the outer balloon 2.

## Description

### [Technical Field]

The present invention relates to a balloon catheter, and more particularly relates to a balloon catheter used for medical treatment of aortic stenosis.

### [Background Art]

When transcatheter aortic valve implantation (TAVI)/transcatheter aortic valve replacement (TAVR) is difficult, balloon aortic valvuloplasty (BAV) is performed. However, the current BAV requires rapid pacing to reduce cardiac output in order to prevent serious complications associated with balloon movement during inflation. However, some patients cannot tolerate such rapid pacing, and frequent rapid pacing can worsen the prognosis. Therefore, there is a need for technology that allows frequent inflation without tachycardia pacing.

A BAV using a drive system in synchronization with an electrocardiogram (hereinafter also referred to as an "electrocardiogram-synchronized BAV" or "electrocardiogram-synchronized aortic valve inflation system") can inflate the aortic valve in accordance with physiological rhythms. Therefore, since it is possible without rapid pacing of frequent inflation, it can be performed on patients who cannot undergo conventional BAV medical treatment, and it further has the advantage of being significantly more effective than existing aortic valve balloons.

Since the electrocardiogram-synchronized BAV can significantly increase the valve orifice area as compared with a conventional inflation method that performs one to three inflations by inflating the aortic valve frequently (not less than at least six times) with a balloon, it has the potential to save patients whose lives could not be saved until now (refer to Non-Patent Literature 1).

The most serious complication in the electrocardiogram-synchronized BAV is air embolism (cerebral infarction) caused by balloon rupture. In order to put the electrocardiogram-synchronized BAV in practical use, it is necessary to reduce such a risk as close to zero as possible.

As a technique of preventing balloon rupture, the inventors have already proposed a balloon catheter including a shaft, an inner balloon, and an outer balloon (refer to Patent Literature 1). This is configured so that an outer balloon is attached to a shaft at a front end side and a rear end side so as to cover an inner balloon, and the elongation rate in the radial direction of the outer balloon is smaller than the elongation rate in the radial direction of the inner balloon. Accordingly, the outer balloon does not expand excessively, thereby avoiding the risk of balloon rupture and enabling safer medical treatment.

However, in the balloon catheter disclosed in Patent Literature 1, the shaft includes an inner balloon connecting lumen communicated from an end surface on a rear end side in a longitudinal direction through the inside of the shaft to the inside of an inner balloon, and an outer balloon connecting lumen communicated to the inside of an outer balloon, and therefore it is not a technology with regard to a balloon catheter having a single inflation lumen.

A well-known technique for using a double balloon in a balloon catheter having a single inflation lumen is a balloon catheter having inner and outer expandable balloons bound to the catheter shaft, the inner balloon having smaller elasticity than the outer balloon (refer to Patent Literature 2).

However, the balloon catheter disclosed in Patent Literature 2 relates to a technology that is intended to be used for cryoballoons. Therefore, when such technology is applied to a BAV, since the inner balloon has smaller elasticity than the outer balloon, there is a problem that the outer balloon is not strong enough and is prone to rupture.

### [Citation List]

### [Patent Literature]

Patent Literature 1: JPA 2011-152181
Patent Literature 2: JPB 7018501

### [Non-Patent Literature]

Non-Patent Literature 1: A. Konishi, "The effect of multiple-inflation balloon aortic valvuloplasty", Heart and Vessels 2020, 35, 1557-1562.

### [Summary of Invention]

### [Technical Problem]

Thus, in the electrocardiogram-synchronized BAV, it is necessary to reduce the risk of air embolism caused by balloon rupture to as close to zero as possible. On the other hand, in BAV, it is necessary to deflate and inflate the balloon quickly, and there is also a need to improve the deflation response of the balloon.

In view of such a situation, an object of a present invention is to provide a balloon catheter including a single inflation lumen, capable of preventing balloon rupture, and having an increased deflation response.

### [Solution to Problem]

To solve the above problems, a dual film balloon catheter according to the present invention is a balloon catheter including a single inflation lumen in a dual film balloon, wherein the dual film balloon includes an outer balloon and an inner balloon, the outer balloon and the inner balloon are bonded to each other at a proximal end of the outer balloon to a proximal end of the inner balloon and at a distal end of the outer balloon to a distal end of the inner balloon, an inner peripheral surface of an inflation part of the outer balloon and an outer peripheral surface of the inflation part of the inner balloon, which are inflated when the dual film balloon expands, are not bonded to each other, and an inner diameter of the outer balloon is larger than an outer diameter of the inner balloon.

The dual film balloon is configured to include the outer and inner balloons that prevent air and other fluids from leaking into the bloodstream even if a hole is formed in the surface of the balloon. Moreover, since the outer balloon and the inner balloon are not bonded to each other at the inflation part, the balloons are not integrated, which has the advantage that the inner balloon is not affected by damage even if the outer balloon is damaged.

Moreover, since the diameters of the outer balloon and the inner balloon are not the same, and the inner diameter of the outer balloon is larger than the outer diameter of the inner balloon, the deflation force of the inner balloon is improved when the balloon is deflated after inflation, thereby obtaining a balloon with sufficient deflation response.

In the dual film balloon catheter according to the present invention, an axial length of the inner balloon is preferably longer than an axial length of the outer balloon. Regarding the sizes of the outer balloon and the inner balloon, not only the diameter, but also the length, the deflation response can be further improved by increasing the axial length of the outer balloon and decreasing the axial length of the inner balloon.

In the dual film balloon catheter according to the present invention, the outer balloon is preferably formed of a material having a higher durometer hardness than that of the inner balloon. The outer balloon is formed of a material harder than that of the inner balloon, thereby effectively preventing the outer balloon from a rupture. Specifically, as materials of the outer balloon, polyether block amide, polyamide, polyurethane, polyethylene, or a mixture thereof are preferably used, and as materials of the inner balloon, polyurethane, or a mixture of polyurethane and polyamide or polyether block amide is preferably used.

In the dual film balloon catheter according to the present invention, the outer balloon and the inner balloon may have different cross-sectional shapes along part of or all of a longitudinal direction. Since the outer balloon is pressed against the inner balloon by external pressure (atmospheric pressure or intracardiac blood pressure), the outer balloon and the inner balloon have different shapes. Accordingly, when the outer balloon is formed of a hard material, the outer balloon will follow the shape of the inner balloon during its inflation process and eventually settle into the shape of the outer balloon. Regarding the cross-sectional shape in this case, for example, when the outer balloon has a substantially cylindrical shape, the inner balloon also has a substantially cylindrical shape at the end of the inflation.

Since a fixation force of a balloon catheter is likely to decrease during the balloon being deflated, it is necessary to prevent a fixing position of the balloon from displacement when the balloon is inflated from its deflated state. Accordingly, for example, by forming the outer balloon ellipsoidal or cylindrical, and forming the inner balloon hourglass, dumbbell, or polygonal such as triangular prism, the outer balloon will be inflated to follow the shape of the inner balloon during the early to middle stages of inflation, strengthening the fixation force to the affected area.

Then, from the middle to the final stage of inflation (maximal inflation state), the outer balloon is already in contact with the affected area and is less likely to be displaced, and therefore, effective inflation of the affected area becomes important rather than increasing the fixation force. Since the outer balloon is formed of a material having a higher durometer hardness than that of the inner balloon, at such stages, the inner balloon will be inflated to follow the shape of the outer balloon. In this way, both the fixation force on the affected area and the effective inflation of the affected area can be achieved.

In the dual film balloon catheter according to the present invention, when a ratio of the outer diameter of the inner balloon to the inner diameter of the outer balloon before assembly as a catheter is not less than 70% and not more than 90% and a ratio of the axial length of the inner balloon to the axial length of the outer balloon is not less than 75% and not more than 90%, space between the inner peripheral surface of the inflation part of the outer balloon and the outer peripheral surface of the inflation part of the inner balloon may be filled with less than 0.1 mL of gas. By setting the amount of gas filling the space between the balloons within such a range, the risk of balloon rupture can be reduced and deflation response can be improved.

An aortic valve inflation system according to the present invention includes: the dual film balloon catheter according to any one of the above-described aspect; and a driving unit configured to charge/discharge gas in synchronization with an electrocardiogram to/from the inflation lumen of the dual film balloon catheter, wherein the inflation lumen automatically repeats deflation and inflation in synchronization with the electrocardiogram. Since the driving unit is configured so that the gas is charged/discharged in synchronization with the electrocardiogram to/from the inflation lumen of the dual film balloon catheter, making it possible to perform frequent inflation without the need for tachycardia pacing, and enabling medical treatment to be performed on patients who could not be treated in the past. Moreover, by providing the dual film balloon catheter, the risk of balloon rupture can be reduced while improving deflation response, resulting in a highly safe and effective system.

As the gas, carbon dioxide gas is preferably used, but other gases may also be used. Alternatively, fluids other than gases may also be used.

### [Advantageous Effects of Invention]

In accordance with the dual film balloon catheter according to the present invention, the balloon catheter including the single inflation lumen have the effect of capable of preventing balloon rupture and having an increased deflation response.

### [Brief Description of Drawings]

FIG. 1 is a cross-sectional image diagram of a dual film balloon catheter of Example 1.
FIG. 2 is an explanatory diagram of the dual film balloon catheter of Example 1.
FIG. 3 is a deflation image diagram of the dual film balloon catheter of Example 1.
FIG. 4 is a functional block diagram of an aortic valve inflation system of Example 1.
FIG. 5 is a usage image diagram of the dual film balloon catheter of Example 1.
FIG. 6 is an explanatory diagram of a gas measurement test between inner and outer balloons.
FIG. 7 is a deflation image diagram of a balloon catheter of a comparative example.

### [Description of Embodiments]

Hereinafter, an example of embodiments of the present invention will be described in detail with reference to the drawings. It is to be noted that the scope of the present invention is not limited to the following embodiments or illustrated examples, and that many modifications and variations can be envisaged.

### [Example 1]

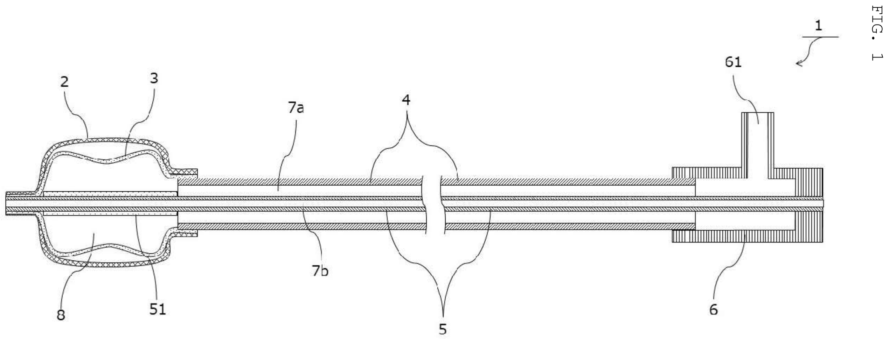

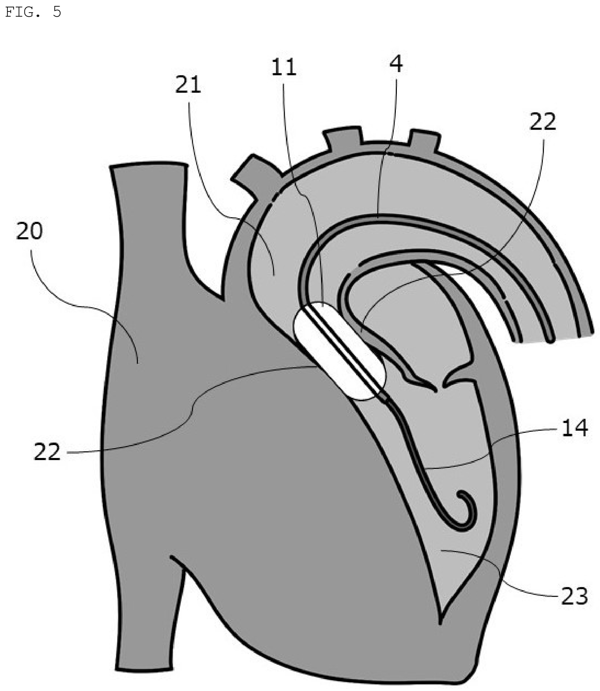

FIG. 1 illustrates a cross-sectional image diagram of a dual film balloon catheter according to Example 1. As illustrated in FIG. 1, the dual film balloon catheter 1 includes an outer balloon 2, an inner balloon 3, an outer tube 4, an inner tube 5, and a grip portion 6. An inner tube 5 is inserted into the outer tube 4, and a tubular lumen 7a is formed between the outer tube 4 and the inner tube 5. Moreover, a tubular lumen 7b is formed in an inner lumina of the inner tube 5 so that a guidewire 14 (refer to FIG. 5) can be inserted thereinto.

An outer balloon 2 and an inner balloon 3 are provided on a front end side of the outer tube 4 and the inner tube 5, and a grip portion 6 is provided on a base end side of the outer tube 4 and the inner tube 5. A through-hole 61 communicating with the tubular lumen 7a is provided in the grip portion 6, carbon dioxide gas is charged/discharged to/from the inflation lumen 8 through the through-hole 61 and the tubular lumen 7a.

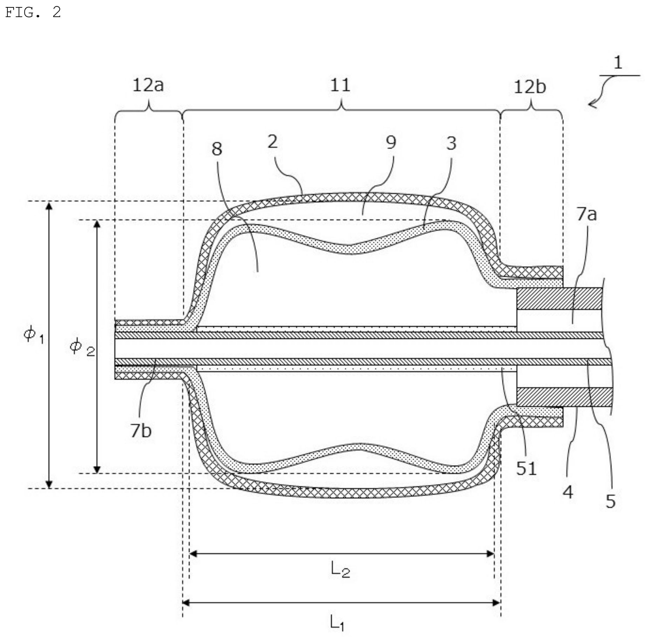

FIG. 2 illustrates an explanatory diagram of the dual film balloon catheter according to the Example 1. As illustrated in FIG. 2, at a bonded part 12a on a front end side, the inner balloon 3 is bonded to the inner tube 5, and the outer balloon 2 is bonded to the inner balloon 3. Moreover, at a bonded part 12b on a base end side, the inner balloon 3 is bonded to the outer tube 4, and the outer balloon 2 is bonded to the inner balloon 3. Consequently, the dual film balloon catheter 1 is configured to be a balloon catheter including a single inflation lumen 8. In addition, the outer balloon 2 and the inner balloon 3 are configured to be not bonded to each other at an inflation part 11.

A reinforcement part 51 is configured to partially reinforce the inner tube 5 positioned at the inflation part 11, and to prevent buckling of the inner tube due to the compressive force of the inner tube generated by the effect of balloon deformation during balloon inflation. Moreover, the reinforcement part 51 is provided with a marker (not illustrated) so as to be easily identified under X-ray radioscopy.

A material of the outer balloon 2 is polyether block amide. A material of the inner balloon 3 is polyurethane.

The outer balloon 2 is formed of a material having a higher durometer hardness than that of the inner balloon 3, a durometer hardness of the outer balloon 2 is 72D, and a durometer hardness of the inner balloon 3 is 80A.

The outer balloon 2 has an ellipsoid shape, but is not limited to such a shape and may have a polygonal shape, such as a substantially triangular prism shape. As illustrated in FIG. 2, the inner balloon 3 differs from the outer balloon 2 and has an hourglass shape having a substantially central portion which is recessed in a longitudinal direction. The hourglass shape improves the fixation force on a cardiac valve. Instead of such a configuration, the inner balloon 3 may have, for example, an ellipsoid shape, without a recess in the central portion in the longitudinal direction.

The maximum outer diameter φ₂ of the inner balloon 3 is set such as to be smaller than the maximum inner diameter φ₁ of the outer balloon 2. Moreover, the length L₂ of the inner balloon 3 in the inflation part 11 is set such as to be shorter than the length L₁ of the outer balloon 2. The advantage of intentionally forming the inner balloon 3 smaller in diameter and length is that a balloon deflation force is generated due to the material after the balloon is inflated, thereby improving the balloon deflation rate.

The bonded structure between the outer balloon 2 and the inner balloon 3 will now be described. In the manufacturing process, the outer balloon 2 and the inner balloon 3 are placed to cover a core (not illustrated) in the order of the inner balloon 3 and the outer balloon 2 and then are bonded to each other at the bonded parts (12a, 12b) of both ends, and then the core is removed. Therefore, the air between the outer balloon 2 and the inner balloon 3 is removed, but a vacuum is not produced, and space 9 is formed between the outer balloon 2 and the inner balloon 3.

The amount of gas present in the space 9 will now be described. Sizes of the outer balloon 2 and the inner balloon 3 before being assembled to the outer tube 4 and the inner tube 5 are preferably such that the inner diameter of the outer balloon 2 is 15 to 30 mm, the outer diameter of the inner balloon 3 is 10.5 to 27 mm, the axial length of the outer balloon 2 is 30 to 50 mm, the axial length of the inner balloon is 22.5 to 45 mm, and the ratio of the outer diameter of the inner balloon 3 to the inner diameter of the outer balloon 2 is not less than 70% and not more than 90%, and the axial length of the inner balloon 3 to the axial length of the outer balloon 2 is not less than 75% and not more than 90%. In the present embodiment, the sizes of the outer balloon 2 and the inner balloon 3 before being assembled to the outer tube 4 and the inner tube 5, the inner diameter of the outer balloon 2 is 20 mm, the outer diameter of the inner balloon 3 is 14 mm, the axial length of the outer balloon 2 is 40 mm, and the axial length of the inner balloon is 30 mm. The sizes of the outer balloon 2 and the inner balloon 3 disclosed herein are sizes obtained by subtracting the sizes of the bonded part 12a and the bonded part 12b.

Under such conditions, a test was performed to measure the amount of gas present between the outer balloon 2 and the inner balloon 3.

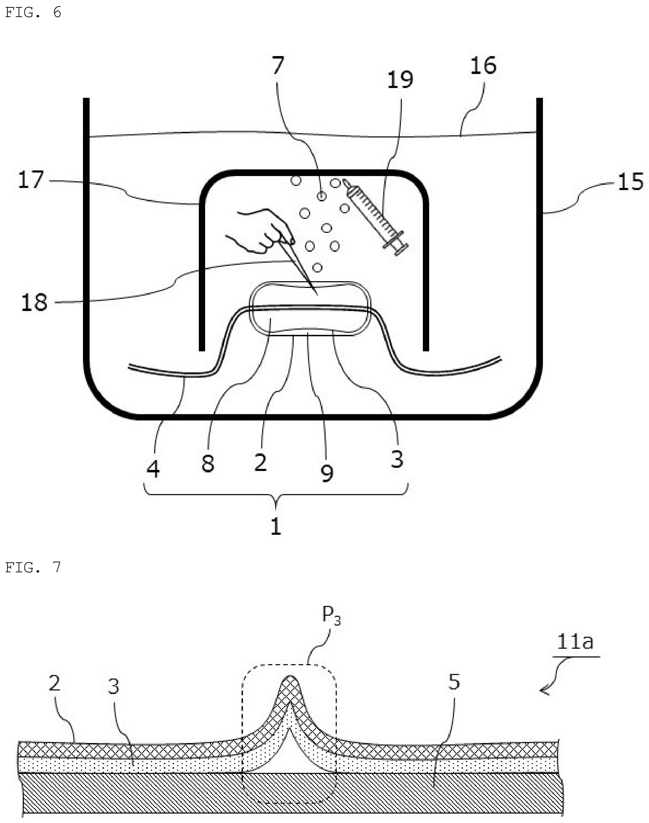

FIG. 6 illustrates an explanatory diagram of a gas measurement test between the inner and outer balloons. In FIG. 6, detailed illustration of the dual film balloon catheter 1 is omitted for the sake of convenience of the description. As illustrated in FIG. 6, first, a water tank 15 is filled up with water 16, and a beaker 17 is submerged in the water tank 15 while being kept free of air. The inside of the inflation lumen 8 of the dual film balloon catheter 1 is filled with water. In this state, the dual film balloon catheter 1 is submerged in the water tank 15, a region of the outer balloon 2 and the inner balloon 3 is covered with the beaker 17, and the outer balloon 2 and the inner balloon 3 are stabbed with a needle 18 to make a hole. In addition, the outer balloon 2 is pressed to squeeze out the gas 7 present in the space 9 to be stored in the beaker 17, and the gas is collected in the syringe 19 of 1 mL to measure the amount of gas. As a consequence, less than 0.1 mL of gas is obtained.

As mentioned above, it is proved that the space between the inner peripheral surface of the inflation part 11 of the outer balloon 2 and the outer peripheral surface of the inflation part 11 of the inner balloon 3 is filled with less than 0.1 mL of gas.

As described above, since the inner balloon 3 is manufactured to have the smaller diameter than that of the outer balloon 2, when the inner balloon 3 is attached to the core, it is placed over the core body while a stretching force is being applied thereto. Consequently, the deflation response when the balloon is deflated can be improved. Moreover, since the outer balloon 2 is pressed against the inner balloon 3 by external pressure, when the outer balloon 2 is formed of a hard material, it will follow the shape of the inner balloon 3 during the inflation process and eventually settle into the shape of the outer balloon 2.

Since the outer balloon 2 and the inner balloon 3 are not bonded to each other at the inflation part 11, the balloons are not integrated, having the advantage that even when the outer balloon 2 is damaged, the inner balloon 3 will not be involved in the damage.

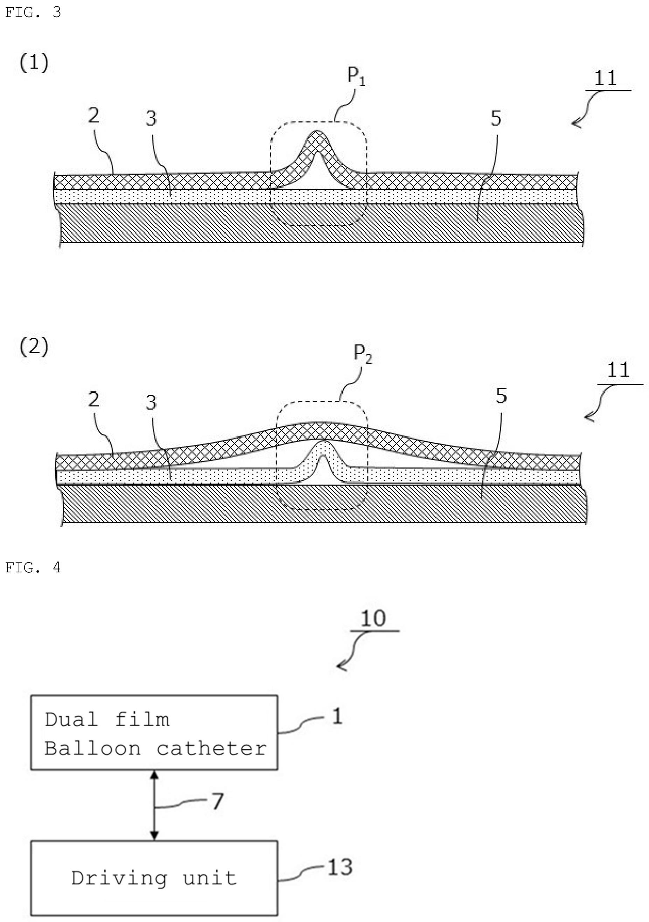

FIG. 3 is a deflation image diagram of the dual film balloon catheter according to Example 1, in which FIG. 3(1) illustrates a state where wrinkles have occurred in the outer balloon, and FIG. 3(2) illustrates a state where wrinkles have occurred in the inner balloon. FIG. 7 illustrates a deflation image diagram of a balloon catheter of a comparative example. Illustration of the reinforcement part 51 is omitted in FIG. 3.

The balloon catheter of the comparative example illustrated in FIG. 7 is configured so that the outer balloon 2 and the inner balloon 3 are bonded to each other over approximately whole surface of the inflation part 11a. Therefore, when the balloons are deflated, the outer balloon 2 and the inner balloon 3, which are integrated with each other, are bent at the same location at the same time, as illustrated in the region P₃ in FIG. 7. The top of the balloon is more susceptible to damage due to the thickness of the integrated outer balloon 2 and inner balloon 3. Moreover, there is a problem that when the outer balloon 2 is damaged, the inner balloon 3 will be involved in the damage.

In contrast, in the balloon catheter 1 according to Example 1, the inflation part 11 is deflated, and even when wrinkles occur in the outer balloon 2 at the region P₁ as illustrated in FIG. 3(1), wrinkles do not necessarily occur in the inner balloon 3 as well. Moreover, as illustrated in FIG. 3(2), even when wrinkles occur in the inner balloon 3 at the region P₂, wrinkles do not necessarily occur in the outer balloon 2 as well.

Therefore, even when wrinkles occur in the outer balloon 2 or in the inner balloon 3, there is an advantage that the thickness of the balloon is less likely to impose a load on the top of the wrinkles. Moreover, the outer balloon 2 and the inner balloon 3 are not integrated with each other and wrinkles occur separately when the balloons are deflated, therefore reducing the risk that when one balloon is damaged, the other balloon will also be damaged at the same time.

Thus, since the outer balloon 2 and the inner balloon 3 are not bonded with each other at the inflation part 11, it is possible to not only prevent each balloon rupture but also to effectively prevent simultaneously rupture of both balloons.

FIG. 4 illustrates a functional block diagram of an aortic valve inflation system according to Example 1. As illustrated in FIG. 4, an aortic valve inflation system 10 includes the dual film balloon catheter 1 and a driving unit 13, and an inflation lumen 8 automatically repeats deflation and inflation in synchronization with an electrocardiogram. The driving unit 13 charges/discharges carbon dioxide gas 7 to/from the inflation lumen 8 of the dual film balloon catheter 1 in synchronization with the electrocardiogram. The driving unit 13 disclosed herein is connected to an electrocardiogram data acquiring means and an electrocardiogram synchronous calculating means, which are not illustrated. The electrocardiogram data acquiring means acquires patient's electrocardiogram data, and the electrocardiogram synchronous calculating means calculates data for electrocardiogram synchronization on the basis of the electrocardiogram data acquired by the electrocardiogram data acquiring means. The driving unit 13 is driven in synchronization with the electrocardiogram on the basis of the calculated data for electrocardiogram synchronization.

FIG. 5 illustrates a usage image diagram of the dual film balloon catheter according to Example 1. As illustrated in FIG. 5, after inserting the guidewire 14 into the left ventricle 23 from the ascending aorta 21 of the heart 20, the dual film balloon catheter 1 guides the inflation part 11 to a position of the aortic valve 22 to perform inflation and deflation.

The aortic valve expansion system 10 can perform frequent inflation without the need for tachycardia pacing, making it possible to medically treat patients who could not be medically treated in the past, and also has advantages such as effectiveness, safety, and simplicity of the procedure.

Using the aortic valve expansion system 10 according to the present embodiment, 500 inflations are performed in a nonclinical testing environment simulating a human. The outer balloon 2 and the inner balloon 3 do not rupture, and the usefulness of the system is confirmed, such as a reduction in the aortic valve pressure differential.

### [Industrial Applicability]

The present invention is useful as a balloon catheter used in the treatment of heart disease and the like.

### [Reference Signs List]

- 1: Dual film balloon catheter;
- 2: Outer balloon;
- 3: Inner balloon;
- 4: Outer tube;
- 5: Inner tube;
- 6: Grip portion;
- 7: Carbon dioxide gas;
- 7a, 7b: Tubular lumen;
- 8: Inflation lumen;
- 9: Space;
- 10: Aortic valve inflation system;
- 11, 11a: Inflation part;
- 12a, 12b: Bonded part;
- 13: Driving unit;
- 14: Guidewire;
- 15: Water tank;
- 16: Water;
- 17: Beaker;
- 18: Needle;
- 19: Syringe;
- 20: Heart;
- 21: Ascending aorta;
- 22: Aortic valve;
- 23: Left ventricle;
- 51: Reinforcement part;
- 61: Through-hole;
- L₁, L₂: Length;
- L₁-L₃: Region;
- φ₁, φ₂: Diameter

## Claims

1. A dual film balloon catheter, being a balloon catheter comprising a single inflation lumen in a dual film balloon, wherein
the dual film balloon comprises an outer balloon and an inner balloon,
the outer balloon and the inner balloon are bonded to each other at a proximal end of the outer balloon to a proximal end of the inner balloon and at a distal end of the outer balloon to a distal end of the inner balloon,
an inner peripheral surface of an inflation part of the outer balloon and an outer peripheral surface of the inflation part of the inner balloon, which are inflated when the dual film balloon expands, are not bonded to each other, and
an inner diameter of the outer balloon is larger than an outer diameter of the inner balloon.

2. The dual film balloon catheter according to claim 1, wherein an axial length of the outer balloon is longer than an axial length of the inner balloon.

3. The dual film balloon catheter according to claim 1, wherein the outer balloon is preferably formed of a material having a higher durometer hardness than that of the inner balloon.

4. The dual film balloon catheter according to claim 3, wherein the outer balloon and the inner balloon may have different cross-sectional shapes along part of or all of a longitudinal direction.

5. The dual film balloon catheter according to claim 2, wherein
when a ratio of the outer diameter of the inner balloon to the inner diameter of the outer balloon before assembly as a catheter is not less than 70% and not more than 90% and a ratio of the axial length of the inner balloon to the axial length of the outer balloon is not less than 75% and not more than 90%,
space between the inner peripheral surface of the inflation part of the outer balloon and the outer peripheral surface of the inflation part of the inner balloon is filled with less than 0.1 mL of gas.

6. An aortic valve inflation system comprising:
the dual film balloon catheter according to any one of claims 1-5; and
a driving unit configured to charge/discharge gas in synchronization with an electrocardiogram to/from the inflation lumen of the dual film balloon catheter, wherein
the inflation lumen automatically repeats deflation and inflation in synchronization with the electrocardiogram.
